# EUROPÄISCHE PATENTSCHRIFT

(11) **EP 1 208 216 B1**
(45) Veröffentlichungstag und Bekanntmachung des Hinweises auf die Patenterteilung: **26.07.2006**
(21) Anmeldenummer: 00956479.0
(22) Anmeldetag: 23.08.2000
(51) Int. Cl.: C12N 15/82, C12N 9/02, C07K 14/415, C12N 1/19, A01H 5/00, C11B 1/00, C11C 1/00

(54) **FETTSÄURE-DESATURASE-GEN AUS PFLANZEN**
FATTY ACID DESATURASE GENE FROM PLANTS
GENE DE DESATURASE D'ACIDE GRAS ISSU DE PLANTES

(30) Priorität: 01.09.1999 DE 19941609
(43) Veröffentlichungstag der Anmeldung: 29.05.2002
(73) Patentinhaber: BASF Aktiengesellschaft, 67056 Ludwigshafen (DE)
(72) Erfinder: FEUSSNER, Ivo, D-06114 Halle (DE); HORNUNG, Ellen, D-06110 Halle (DE); FRITSCHE, Kathrin, D-6811 HN Arnhem (NL); PEITZSCH, Nicola, D-06484 Quedlinburg (DE); RENZ, Andreas, D-67117 Limburgerhof (DE)
(86) Internationale Anmeldenummer: PCT/EP2000/008222
(87) Internationale Veröffentlichungsnummer: WO 2001/016362

(56) Entgegenhaltungen:
- EP-A- 0 945 514
- WO-A-00/11176
- WO-A-01/12800
- WO-A-94/11516
- WO-A-97/35987
- WO-A-97/37033
- WO-A-98/46762
- WO-A-98/56922
- GB-A- 2 323 031
- US-A- 5 850 026
- MEIER ZU BEERENTRUP H ET AL: "CALENDULA AND CORIANDRUM NEW POTENTIAL OIL CROPS FOR INDUSTRIAL USES" FETT WISSENSCHAFT TECHNOLOGIE, Bd. 89, Nr. 6, 1987, Seiten 227-230, XP002162405 ISSN: 0931-5985
- CROMBIE L ET AL: "THE BIOSYNTHESIS OF CALENDIC-ACID OCTADECA-8E 10E 12Z-TRIENOIC-ACID BY DEVELOPING MARIGOLD SEEDS ORIGINS OF E E Z AND Z E Z CONJUGATED TRIENE ACIDS IN HIGHER PLANTS" JOURNAL OF THE CHEMICAL SOCIETY PERKIN TRANSACTIONS I, Nr. 11, 1985, Seiten 2425-2434, XP000973290 ISSN: 0300-922X
- LIU LINSEN ET AL: "In vivo studies of the biosynthesis of alpha-eleostearic acid in the seed of Momordica charantia L." PLANT PHYSIOLOGY (ROCKVILLE), Bd. 113, Nr. 4, 1997, Seiten 1343-1349, XP000973396 ISSN: 0032-0889
- GUNSTONE, F.D., ET AL.: "The LIpid Handbook: second edition" 1994 , CHAPMAN & HALL , LONDON XP002162924 Seite 7 -Seite 8 Seite 51 Seite 109
- DATABASE BIOSIS [Online] BIOSCIENCES INFORMATION SERVICE, PHILADELPHIA, PA, US; 1998 DHAR P ET AL: "Nutritional characteristics of oil containing conjugated octadecatrienoic fatty acid." Database accession no. PREV199900002659 XP002162410 & ANNALS OF NUTRITION & METABOLISM, Bd. 42, Nr. 5, 1998, Seiten 290-296, ISSN: 0250-6807
- FRITSCHE KATHRIN ET AL: "Isolation and characterization of a calendic acid producing (8,11)-linoleoyl desaturase." FEBS LETTERS, Bd. 462, Nr. 3, 3. Dezember 1999 (1999-12-03), Seiten 249-253, XP000941865 ISSN: 0014-5793 -& DATABASE EMBL [Online] ACCESSION NO: AJ245938, 22. Dezember 1999 (1999-12-22) FUESSNER, I.: "Calendula officinalis partial mRNA for (8,11)-linoleoyl desaturase (des8.11gene)" XP002162411
- CAHOON EDGAR B ET AL: "Biosynthetic origin of conjugated double bonds: Production of fatty acid components of high-value drying oils in transgenic soybean embryos." PROCEEDINGS OF THE NATIONAL ACADEMY OF SCIENCES OF THE UNITED STATES, Bd. 96, Nr. 22, 26. Oktober 1999 (1999-10-26), Seiten 12935-12940, XP002162407 Oct. 26, 1999 ISSN: 0027-8424
- CAHOON, B, ET AL.: "Formation of Conjugated Delta 8,Delta 10-Double Bonds by Delta 12-Oleic-acid Desaturase-related Enzymes. BIOSYNTHETIC ORIGIN OF CALENDIC ACID" THE JOURNAL OF BIOLOGICAL CHEMISTRY, Bd. 276, 2001, Seiten 2637-2643, XP000990245
- HAMBERG MATS: "Metabolism of 6,9,12-octadecatrienoic acid in the red alga Lithothamnion corallioides: Mechanism of formation of a conjugated tetraene fatty acid." BIOCHEMICAL AND BIOPHYSICAL RESEARCH COMMUNICATIONS, Bd. 188, Nr. 3, 1992, Seiten 1220-1227, XP000941893 ISSN: 0006-291X

### SEQUENZPROTOKOLL

<110> Institut für Pflanzenbiochemie
<120> Fettsäure-Desaturase-Gen aus Pflanzen
<130> Sequenz_Desaturase
<140> 50669
   <141> 1999-08-31
<160> 2
<170> PatentIn Vers. 2.0
<210> 1
   <211> 1285
   <212> DNA
   <213> Calendula officinalis
<220>
   <221> CDS
   <222> (42)..(1175)
<400> 1
   aaaagctcac ttctctgtga gggtaattat atatcaacaa c atg ggt gct ggt ggt 56
                                                 Met Gly Ala Gly Gly
                                                  1               5
   cgg atg tcg gat cca tct gag gga aaa aac atc ctt gaa cgt gtg cca   104
   Arg Met Ser Asp Pro Ser Glu Gly Lys Asn Ile Leu Glu Arg Val Pro
                   10                  15                  20
   gtc gat cca ccg ttc acg tta agc gat ctg aag aaa gcg att cct acc   152
   Val Asp Pro Pro Phe Thr Leu Ser Asp Leu Lys Lys Ala Ile Pro Thr
               25                  30                  35
   cat tgc ttt gag cga tct gtc atc cgg tca tca tac tat gtt gtt cat   200
   His Cys Phe Glu Arg Ser Val Ile Arg Ser Ser Tyr Tyr Val Val His
           40                  45                  50
   gat ctc att gtt gcc tat gtc ttc tac tac ctt gca aac acg tat atc   248
   Asp Leu Ile Val Ala Tyr Val Phe Tyr Tyr Leu Ala Asn Thr Tyr Ile
       55                  60                  65
   cct ctt att cct aca cct ctg gct tac cta gca tgg ccc gtt tac tgg   296
   Pro Leu Ile Pro Thr Pro Leu Ala Tyr Leu Ala Trp Pro Val Tyr Trp
   70                  75                  80                  85
   ttt tgt caa gct agc atc ctc acc ggc ctc tgg gtc atc ggt cac gaa   344
   Phe Cys Gln Ala Ser Ile Leu Thr Gly Leu Trp Val Ile Gly His Glu
                       90                  95                  100
   tgt ggt cac cat gca ttt agc gac tac cag ttg att gat gac att gtt     392
   Cys Gly His His Ala Phe Ser Asp Tyr Gln Leu Ile Asp Asp Ile Val
               105                 110                 115
   gga ttc gtg ctc cat tcg gct ctc ctc acc ccg tat ttc tct tgg aaa     440
   Gly Phe Val Leu His Ser Ala Leu Leu Thr Pro Tyr Phe Ser Trp Lys
           120                 125                 130
   tat agc cac agg aat cac cac gcc aac aca aat tca ctc gat aac gat     488
   Tyr Ser His Arg Asn His His Ala Asn Thr Asn Ser Leu Asp Asn Asp
       135                 140                 145
   gaa gtt tac att cct aaa cgt aag tcg aag gtc aag att tat tcc aaa     536
   Glu Val Tyr Ile Pro Lys Arg Lys Ser Lys Val Lys Ile Tyr Ser Lys
   150                 155                 160                 165
   ctt ctt aac aat cca ccc ggg cga gtg ttc act ttg gtg ttt cgg ttg     584
   Leu Leu Asn Asn Pro Pro Gly Arg Val Phe Thr Leu Val Phe Arg Leu
                   170                 175                 180
   act tta gga ttt ccg tta tac ctc tta act aat atc tcg ggc aag aaa     632
   Thr Leu Gly Phe Pro Leu Tyr Leu Leu Thr Asn Ile Ser Gly Lys Lys
               185                 190                 195
   tac ggg agg ttt gcc aac cac ttt gat ccc atg agt cca att ttc aac     680
   Tyr Gly Arg Phe Ala Asn His Phe Asp Pro Met Ser Pro Ile Phe Asn
           200                 205                 210
   gat cgt gaa cgc gtt caa gtt ttg cta tcc gat ttc ggt ctt ctc gct     728
   Asp Arg Glu Arg Val Gln Val Leu Leu Ser Asp Phe Gly Leu Leu Ala
       215                 220                 225
   gta ttt tat gca atc aag ctt ctt gta gca gca aaa ggg gca gct tgg     776
   Val Phe Tyr Ala Ile Lys Leu Leu Val Ala Ala Lys Gly Ala Ala Trp
   230                 235                 240                 245
   gta atc aac atg tac gca att cca gta cta ggt gta agc gtg ttc ttc     824
   Val Ile Asn Met Tyr Ala Ile Pro Val Leu Gly Val Ser Val Phe Phe
                   250                 255                 260
   gtt ttg atc aca tat ttg cac cac acc cat ctc tca ctc cct cat tat     872
   Val Leu Ile Thr Tyr Leu His His Thr His Leu Ser Leu Pro His Tyr
               265                 270                 275
   gat tca acc gaa tgg aac tgg atc aaa ggc gcc tta tca aca atc gat     920
   Asp Ser Thr Glu Trp Asn Trp Ile Lys Gly Ala Leu Ser Thr Ile Asp
           280                 285                 290
   agg gat ttc ggg ttc ctg aat cgg gtt ttc cac gac gtt aca cac act     968
   Arg Asp Phe Gly Phe Leu Asn Arg Val Phe His Asp Val Thr His Thr
       295                 300                 305
   cac gtc ttg cat cat ttg atc tca tac att cca cat tat cat gca aag   1016
   His Val Leu His His Leu Ile Ser Tyr Ile Pro His Tyr His Ala Lys
   310                 315                 320                 325
   gaa gca agg gat gca atc aag cca gtg ttg ggc gag tac tat aaa atc   1064
   Glu Ala Arg Asp Ala Ile Lys Pro Val Leu Gly Glu Tyr Tyr Lys Ile
                   330                 335                 340
   gac agg act cca att ttc aaa gca atg tat aga gag gct aag gaa tgc   1112
   Asp Arg Thr Pro Ile Phe Lys Ala Met Tyr Arg Glu Ala Lys Glu Cys
               345                 350                 355
   atc tac atc gag ccc gat gag gat agc gag cac aaa ggt gtg ttc tgg   1160
   Ile Tyr Ile Glu Pro Asp Glu Asp Ser Glu His Lys Gly Val Phe Trp
           360                 365                 370
   tac cac aag atg taa tcaaaaaggt gtatgtcaat gcaattgtat gcttaattaa   1215
   Tyr His Lys Met
       375
   gttgttaaac tttctattcc gtgtaataaa ttatcattaa gagaaaaaaa aaaaaaaaaa 1275
   aaaaaaaaaa                                                        1285
<210> 2
   <211> 377
   <212> PRT
   <213> Calendula officinalis
<400> 2
   Met Gly Ala Gly Gly Arg Met Ser Asp Pro Ser Glu Gly Lys Asn Ile
    1               5                  10                  15
   Leu Glu Arg Val Pro Val Asp Pro Pro Phe Thr Leu Ser Asp Leu Lys
               20                  25                  30
   Lys Ala Ile Pro Thr His Cys Phe Glu Arg Ser Val Ile Arg Ser Ser
           35                  40                  45
   Tyr Tyr Val Val His Asp Leu Ile Val Ala Tyr Val Phe Tyr Tyr Leu
       50                  55                  60
   Ala Asn Thr Tyr Ile Pro Leu Ile Pro Thr Pro Leu Ala Tyr Leu Ala
   65                  70                  75                  80
   Trp Pro Val Tyr Trp Phe Cys Gln Ala Ser Ile Leu Thr Gly Leu Trp
                   85                  90                  95
   Val Ile Gly His Glu Cys Gly His His Ala Phe Ser Asp Tyr Gln Leu
   100 105 110
   Ile Asp Asp Ile Val Gly Phe Val Leu His Ser Ala Leu Leu Thr Pro
   115 120 125
   Tyr Phe Ser Trp Lys Tyr Ser His Arg Asn His His Ala Asn Thr Asn
   130 135 140
   Ser Leu Asp Asn Asp Glu Val Tyr Ile Pro Lys Arg Lys Ser Lys Val
   145 150 155 160
   Lys Ile Tyr Ser Lys Leu Leu Asn Asn Pro Pro Gly Arg Val Phe Thr
   165 170 175
   Leu Val Phe Arg Leu Thr Leu Gly Phe Pro Leu Tyr Leu Leu Thr Asn
   180 185 190
   Ile Ser Gly Lys Lys Tyr Gly Arg Phe Ala Asn His Phe Asp Pro Met
   195 200 205
   Ser Pro Ile Phe Asn Asp Arg Glu Arg Val Gln Val Leu Leu Ser Asp
   210 215 220
   Phe Gly Leu Leu Ala Val Phe Tyr Ala Ile Lys Leu Leu Val Ala Ala
   225 230 235 240
   Lys Gly Ala Ala Trp Val Ile Asn Met Tyr Ala Ile Pro Val Leu Gly
   245 250 255
   Val Ser Val Phe Phe Val Leu Ile Thr Tyr Leu His His Thr His Leu
   260 265 270
   Ser Leu Pro His Tyr Asp Ser Thr Glu Trp Asn Trp Ile Lys Gly Ala
   275 280 285
   Leu Ser Thr Ile Asp Arg Asp Phe Gly Phe Leu Asn Arg Val Phe His
   290 295 300
   Asp Val Thr His Thr His Val Leu His His Leu Ile Ser Tyr Ile Pro
   305 310 315 320
   His Tyr His Ala Lys Glu Ala Arg Asp Ala Ile Lys Pro Val Leu Gly
   325 330 335
   Glu Tyr Tyr Lys Ile Asp Arg Thr Pro Ile Phe Lys Ala Met Tyr Arg
   340 345 350
   Glu Ala Lys Glu Cys Ile Tyr Ile Glu Pro Asp Glu Asp Ser Glu His
   355 360 365
   Lys Gly Val Phe Trp Tyr His Lys Met
   370 375

## Beschreibung

Die vorliegende Erfindung betrifft ein Verfahren zur Herstellung von ungesättigten oder gesättigten Fettsäuren sowie ein Verfahren zur Herstellung von Triglyceriden mit einem erhöhten Gehalt an ungesättigten oder gesättigten Fettsäuren.

Die Erfindung betrifft weiterhin eine Nukleinsäuresequenz; ein Nukleinesäurekonstrukt, einen Vektor und Organismen enthaltend mindestens eine Nukleinsäuresequenz bzw. ein Nukleinsäurekonstrukt. Außerdem betrifft die Erfindung gesättigte oder ungesättigte Fettsäuren sowie Triglyceride mit einem erhöhten Gehalt an ungesättigten oder gesättigten Fettsäuren und deren Verwendung.

Fettsäuren und Triglyceride haben eine Vielzahl von Anwendungen in der Lebensmittelindustrie, der Tierernährung, der Kosmetik und im Pharmabereich. Je nachdem ob es sich um freie gesättigte oder ungesättigte Fettsäuren oder um Triglyceride mit einem erhöhten Gehalt an gesättigten oder ungesättigten Fettsäuren handelt, sind sie für die unterschiedlichsten Anwendungen geeignet, so werden beispielsweise mehrfach ungesättigte Fettsäuren Babynahrung zur Erhöhung des Nährwertes zugesetzt. Hauptsächlich werden die verschiedenen Fettsäuren und Triglyceride aus Mikroorganismen wie Mortierella oder aus Öl-produzierenden Pflanzen wie Soja, Raps, Sonnenblume und weiteren gewonnen, wobei sie in der Regel in Form ihrer Triacylglyceride anfallen. Sie werden aber auch vorteilhaft aus Tieren wie Fischen gewonnen. Die freien Fettsäuren werden vorteilhaft durch Verseifung hergestellt.

Je nach Anwendungszweck sind Öle mit gesättigten oder ungesättigten Fettsäuren bevorzugt, so sind z.B. in der humanen Ernährung Lipide mit ungesättigten Fettsäuren speziell mehrfach ungesättigten Fettsäuren bevorzugt, da sie einen positiven Einfluß auf den Cholesterinspiegel im Blut und damit auf die Möglichkeit einer Herzerkrankung haben. Sie finden in verschiedenen diätischen Lebensmitteln oder Medikamenten Anwendung.

Besonders wertvolle und gesuchte ungesättigte Fettsäuren sind die sogenannten konjugierten ungesättigten Fettsäuren wie die konjugierte Linolsäure. Für konjugierte Fettsäuren sind eine Reihe positiver Effekte nachgewiesen worden, so reduziert die Verabreichung von konjugierter Linolsäure das Körperfett in Mensch und Tier bzw. erhöht den Futterumsatz in Körpergewicht bei Tieren (WO 94/16690, WO 96/06605, WO 97/46230, WO 97/46118). Durch Gabe von konjugierter Linolsäure lassen sich auch beispielsweise Allergien (WO 97/32008) oder Krebs positiv (Banni et al., Carcinogenesis, Vol. 20, 1999: 1019 - 1024, Thompson et al., Cancer, Res., Vol. 57, 1997: 5067 - 5072) beeinflussen.

Die chemische Herstellung konjugierter Fettsäuren beispielsweise Calendulasäure oder konjugierter Linolsäure wird in US 3,356,699 und US 4,164,505 beschrieben. Calendulasäure kommt natürlich in Calendula officinalis vor (U1'chenko et al., Chemistry of Natural Compounds, 34, 1998: 272 - 274). Konjugierte Linolsäure findet sich beispielsweise in Rindfleisch (Chin et al., Journal of Food Composition and Analysis, 5, 1992: 185 - 197). Biochemische Untersuchungen zur Synthese von Calendulasäure sind in Crombie et al., J. Chem. Soc. Chem. Commun., 15, 1984: 953 - 955 und J. Chem. Soc. Perkin Trans., 1, 1985: 2425 - 2434 zu finden.

Aufgrund ihrer positiven Eigenschaften hat es in der Vergangenheit nicht an Ansätzen gefehlt Gene, die an der Synthese von Fettsäuren bzw. Triglyceriden beteiligt sind, für die Herstellung von Ölen in verschiedenen Organismen mit geändertem Gehalt an ungesättigten Fettsäuren verfügbar zu machen. So wird in WO 91/13972 und seinem US-Äquivalent eine Δ-9-Desaturase beschrieben. In WO 93/11245 wird eine Δ-15-Desaturase; in WO 94/11516 wird eine Δ-12-Desaturase beansprucht. Δ-6-Desaturasen werden in WO 93/06712 und WO 96/21022 beschrieben. Weitere Desaturasen werden beispielsweise in EP-A-0 550 162, WO 94/18337, WO 97/30582, WO 97/21340, WO 95/18222, EP-A-0 794 250, Stukey et al., J. Biol. Chem., 265, 1990: 20144 - 20149, Wada et al., Nature 347, 1990: 200-203 oder Huang et al., Lipids 34, 1999: 649 - 659 beschrieben. Die biochemische Charakterisierung der verschiedenen Desaturasen ist jedoch bisher nur unzureichend erfolgt, da die Enzyme als membrangebundene Proteine nur sehr schwer zu isolieren und charakterisieren sind (McKeon et al., Methods in Enzymol. 71, 1981: 12141 - 12147, Wang et al., Plant Physiol. Biochem., 26, 1988: 777 - 792).

In Hefen konnte sowohl eine Verschiebung des Fettsäurespektrums zu ungesättigten Fettsäuren hin als auch eine Steigerung der Produktivität nachgewiesen werden (siehe Huang et al., Lipids 34, 1999: 649 - 659, Napier et al., Biochem. J., Vol. 330, 1998: 611 - 614). Die Expression der verschiedenen Desaturasen in transgenen Pflanzen zeigte allerdings nicht den gewünschten Erfolg. Eine Verschiebung des Fettsäurespektrum zu ungesättigten Fettsäuren hin konnte gezeigt werden, gleichzeitig zeigte sich aber, daß die Syntheseleistung der transgenen Pflanzen stark nachließ, das heißt gegenüber den Ausgangspflanzen konnten nur geringere Mengen an Ölen isoliert werden.

Nach wie vor besteht daher ein großer Bedarf an neuen Genen, die für Enzyme kodieren, die an der Biosynthese ungesättigter Fettsäuren beteiligt sind und es ermöglichen diese und speziell konjugierte ungesättigte Fettsäuren zu synthetisieren und in einem technischen Maßstab herzustellen.

Es bestand daher die Aufgabe weitere Desaturasen für die Synthese ungesättigter konjugierter Fettsäuren zur Verfügung zu stellen. Diese Aufgabe wurde durch eine isolierte Nukleinsäuresequenz gelöst, die für ein Polypeptid mit Desaturaseaktivität codiert, ausgewählt aus der Gruppe:
a) einer Nukleinsäuresequenz mit der in SEQ ID NO: 1 dargestellten Sequenz,
b). Nukleinsäuresequenzen, die sich als Ergebnis des degenerierten genetischen Codes von der in SEQ ID NO: 1 dargestellten Nukleinsäuresequenz ableiten,
c) Derivate der in SEQ ID NO: 1 dargestellten Nukleinsäuresequenz, die für Polypeptide mit der in SEQ ID NO: 2 dargestellten Aminosäuresequenzen codieren und mindestens 75 % Homologie auf Aminosäureebene aufweisen, ohne daß die enzymatische Wirkung der Polypeptide wesentlich reduziert ist.

Unter Derivate(n) sind beispielsweise funktionelle Homologe des von SEQ ID NO: 1 kodierten Enzyms oder dessen enzymatischer Aktivität, das heißt Enzyme, die dieselben enzymatischen Reaktionen wie das von SEQ ID NO:1 kodierte Enzym katalysieren, zu verstehen. Diese Gene ermöglichen ebenfalls eine vorteilhafte Herstellung ungesättigter konjugierte Fettsäuren. Unter ungesättigten Fettsäuren sind im folgenden einfach und mehrfach ungesättigte Fettsäuren zu verstehen, deren Doppelbindungen konjugiert oder nicht konjugiert sein können. Die in SEQ ID NO:1 genannte Sequenz kodiert für eine neue unbekannte Desaturase, die an der Synthese von Calendulasäure in Calendula officinalis beteiligt ist. Das Enzym setzt (9Z,12Z)Octadecadien/Linolsäure zu (8E,10E,12Z) Octadecakonjutrien/Calendulasäure um. Im folgenden wird sie als Calendulasäure-Desaturase bezeichnet.

Die erfindungsgemäße Nukleinsäuresequenz oder Fragmente davon können vorteilhaft zur Isolierung weiterer genomischer Sequenzen über Homologiescreening verwendet werden.

Die genannten Derivate lassen sich beispielsweise aus anderen eukaryontischen Organismen wie Pflanzen wie Calendula stellata, Osteospermum spinescens oder Osteospermum hyoseroides, Algen, Protozoen wie Dinoflagellaten oder Pilze isolieren.

Weiterhin sind unter Derivaten bzw. funktionellen Derivaten der in SEQ ID No.1 genannten Sequenz beispielsweise Nukleinsäuren zu verstehen, die sich über Hybridisierung bei 42°C in 5 × SSC und 50% Formamid isolieren lassen.

Die von der genannten Nukleinsäure abgeleitete Aminosäuresequenz ist Sequenz SEQ ID No.2 zu entnehmen. Allelvarianten umfassen insbesondere funktionelle Varianten, die durch Deletion, Insertion oder Substitution von Nukleotiden aus der in SEQ ID No.1 dargestellten Sequenz erhältlich sind, wobei die enzymatische Aktivität der abgeleiteten synthetisierten Proteine erhalten bleibt.

Solche DNA-Sequenzen lassen sich ausgehend von der in SEQ ID NO:1 beschriebenen DNA-Sequenz oder Teilen dieser Sequenzen, beispielsweise mit üblichen Hybridisierungsverfahren oder der PCR-Technik aus anderen Eukaryonten wie oben genannt isolieren. Diese DNA-Sequenzen hybridisieren unter Standardbedingungen mit den genannten Sequenzen. Zur Hybrisierung werden vorteilhaft kurze Oligonukleotide beispielsweise der konservierten Bereiche, die über Vergleiche mit anderen Desaturasegenen in dem Fachmann bekannterweise ermittelt werden können, verwendet. Es können aber auch längere Fragmente der erfindungsgemäßen Nukleinsäuren oder die vollständigen Sequenzen für die Hybridisierung verwendet werden. Je nach der verwendeten Nukleinsäure: Oligonukleotid, längeres Fragment oder vollständige Sequenz oder je nachdem welche Nukleinsäureart DNA oder RNA für die Hybridisierung verwendet werden, variieren diese Standardbedingungen. So liegen beispielsweise die Schmelztemperaturen für DNA:DNA-Hybride ca 10 °C niedriger als die von DNA:RNA-Hybriden gleicher Länge.

Unter Standardbedingungen sind beispielsweise je nach Nukleinsäure Temperaturen zwischen 42 und 58 °C in einer wäßrigen Pufferlösung mit einer Konzentration zwischen 0,1 bis 5 x SSC (1 X SSC = 0,15 M NaCl, 15 mM Natriumcitrat, pH 7,2) oder zusätzlich in Gegenwart von 50% Formamid wie beispielsweise 42 °C in 5 x SSC, 50% Formamid zu verstehen. Vorteilhafterweise liegen die Hybridisierungsbedingungen für DNA:DNA-Hybride bei 0,1 x SSC und Temperaturen zwischen etwa 20 °C bis 45 °C, bevorzugt zwischen etwa 30 °C bis 45 °C. Für DNA:RNA-Hybride liegen die Hybridisierungsbedingungen vorteilhaft bei 0,1 x SSC und Temperaturen zwischen etwa 30 °C bis 55 °C, bevorzugt zwischen etwa 45 °C bis 55 °C. Diese angegebenen Temperaturen für die Hybridisierung sind beispielhaft kalkulierte Schmelztemperaturwerte für eine Nukleinsäure mit einer Länge von ca. 100 Nukleotiden und einem G + C-Gehalt von 50 % in Abwesenheit von Formamid. Die experimentellen Bedingungen für die DNA-Hybridisierung sind in einschlägigen Lehrbüchern der Genetik wie beispielsweise Sambrook et al., "Molecular Cloning", Cold Spring Harbor Laboratory, 1989, beschrieben und lassen sich nach dem Fachmann bekannten Formeln beispielsweise abhängig von der Länge der Nukleinsäuren, der Art der Hybride oder dem G + C-Gehalt berechnen. Weitere Informationen zur Hybridisierung kann der Fachmann folgenden Lehrbüchern entnehmen: Ausubel et al. (eds), 1985, Current Protocols in Molecular Biology, John Wiley & Sons, New York; Hames and Higgins (eds), 1985, Nucleic Acids Hybridization: A Practical Approach, IRL Press at Oxford University Press, Oxford; Brown (ed), 1991, Essential Molecular Biology: A Practical Approach, IRL Press at Oxford University Press, Oxford.

Für eine optimale Expression heterologer Gene in Organismen ist es vorteilhaft die Nukleinsäuresequenzen entsprechend des im Organismus verwendeten spezifischen "codon usage" zu verändern. Der "codon usage" läßt sich anhand von Computerauswertungen anderer, bekannter Gene des betreffenden Organismus leicht ermitteln.

Vorteilhaft kann das Calendulasäure-Desaturase-Gen im erfindungsgemäßen Verfahren mit weiteren Genen der Fettsäurebiosynthese kombiniert werden.

Unter den erfindungsgemäßen Aminosäuresequenzen sind Proteine zu verstehen, die eine in SEQ ID NO: 2 dargestellte Aminosäuresequenz oder eine daraus durch Substitution, Inversion, Insertion oder Deletion von einem oder mehreren Aminosäureresten erhältliche Sequenz enthalten, wobei die enzymatische Aktivität des in SEQ ID NO: 2 dargestellten Proteins erhalten bleibt bzw. nicht wesentlich reduziert wird. Unter nicht wesentlich reduziert sind alle Enzyme zu verstehen, die noch mindestens 10 %, bevorzugt 20 %, besonders bevorzugt 30 % der enzymatischen Aktivität des Ausgangsenzyms aufweisen. Dabei können beispielsweise bestimmte Aminosäuren durch solche mit ähnlichen physikochemischen Eigenschaften (Raumerfüllung, Basizität, Hydrophobizität etc.) ersetzt werden. Beispielsweise werden Argininreste gegen Lysinreste, Valinreste gegen Isoleucinreste oder Asparaginsäurereste gegen Glutaminsäurereste ausgetauscht. Es können aber auch ein oder mehrere Aminosäuren in ihrer Reihenfolge vertauscht, hinzugefügt oder entfernt werden, oder es können mehrere dieser Maßnahmen miteinander kombiniert werden.

Unter dem erfindungsgemäßen Nukleinsäurekonstrukt oder -fragment ist die in SEQ ID NO: 1 genannte Sequenz, Sequenzen, die sich als Ergebnis des degenerierten genetischen Codes von der in Seq ID No:1 dargestellten Nukleinsäuresequenz ableiten, zu verstehen, die mit einem oder mehreren Regulationssignalen vorteilhafterweise zur Erhöhung der Genexpression funktionell verknüpft wurden. Beispielsweise handelt es sich bei diesen regulatorischen Sequenzen um Sequenzen an die Induktoren oder Repressoren binden und so die Expression der Nukleinsäure regulieren. Zusätzlich zu diesen neuen Regulationssequenzen oder anstelle dieser Sequenzen kann die natürliche Regulation dieser Sequenzen vor den eigentlichen Strukturgenen noch vorhanden sein und gegebenenfalls genetisch verändert worden sein, so daß die natürliche Regulation ausgeschaltet und die Expression der Gene erhöht wurde. Das Genkonstrukt kann aber auch einfacher aufgebaut sein, das heißt es wurden keine zusätzlichen Regulationssignale vor die Sequenz oder deren Derivate inseriert und der natürliche Promotor mit seiner Regulation wurde nicht entfernt. Stattdessen wurde die natürliche Regulationssequenz so mutiert, daß keine Regulation mehr erfolgt und die Genexpression gesteigert wird. Diese veränderten Promotoren können auch allein vor das natürliche Gen zur Steigerung der Aktivität gebracht werden. Das Genkonstrukt kann außerdem vorteilhafterweise auch eine oder mehrere sogenannte "enhancer Sequenzen" funktionell verknüpft mit dem Promotor enthalten, die eine erhöhte Expression der Nukleinsäuresequenz ermöglichen. Auch am 3'-Ende der DNA-Sequenzen können zusätzliche vorteilhafte Sequenzen inseriert werden wie weitere regulatorische Elemente oder Terminatoren. Das Calendulasäure-Desaturase-Gen kann in einer oder mehreren Kopien im Genkonstrukt enthalten sein.

Vorteilhafte Regulationssequenzen für das erfindungsgemäße Verfahren sind beispielsweise in Promotoren wie cos-, tac-, trp-, tet-, trp-tet-, lpp-, lac-, lpp-lac-, lacI^{q-}, T7-, T5-, T3-, gal-, trc-, ara-, SP6-, λ-P_{R}- oder im λ-P_{L}-Promotor enthalten, die vorteilhafterweise in gram-negativen Bakterien Anwendung finden. Weitere vorteilhafte Regulationssequenzen sind beispielsweise in den gram-positiven Promotoren amy und SPO2, in den Hefe- oder Pilzpromotoren ADC1, MFα, AC, P-60, CYC1, GAPDH, TEF, rp28, ADH oder in den Pflanzenpromotoren wie CaMV/35S [Franck et al., Cell 21(1980) 285-294], PRP1 [Ward et al., Plant.Mol. Biol.22(1993)], SSU, OCS, lib4, STLS1, B33, nos oder im Ubiquitin-Promotor enthalten. Weitere vorteilhafte Pflanzenpromotoren sind beispielsweise ein durch Benzensulfonamid-induzierbarer (EP 388186), ein durch Tetrazyklin-induzierbarer (Gatz et al., (1992) Plant J. 2,397-404), ein durch Abscisinsäure-induzierbarer (EP335528) bzw. ein durch Ethanol- oder Cyclohexanon-induzierbarer (WO9321334) Promotor. Weitere Pflanzenpromotoren sind beispielsweise der Promotor der cytosolischen FBPase aus Kartoffel, der ST-LSI Promotor aus Kartoffel (Stockhaus et al., EMBO J. 8 (1989) 2445-245), der Promotor der Phosphoribosylpyrophosphat Amidotransferase aus Glycine max (siehe auch Genbank Accession Nummer U87999) oder ein Nodien-spezifischen Promotor wie in EP 249676 beschrieben. Vorteilhaft sind insbesondere solche pflanzliche Promotoren, die die Expression in Geweben oder Pflanzenteilen sicherstellen, in denen die Fettbiosynthese bzw. dessen Vorstufen stattfindet. Insbesondere zu nennen sind Promotoren, die eine samenspezifische Expression gewährleisten wie beispielsweise der usp-Promotor, der LEB4-Promotor, der Phaseolin-Promotor oder der Napin-Promotor.

Prinzipiell können alle natürlichen Promotoren mit ihren Regulationssequenzen wie die oben genannten für das erfindungsgemäße Verfahren verwendet werden. Darüberhinaus können auch synthetische Promotoren vorteilhaft verwendet werden.

Im Nukleinsäurefragment (= Genkonstrukt, Nukleinsäurekonstrukt) können wie oben beschrieben noch weitere Gene, die in die Organismen eingebracht werden sollen, enthalten sein. Diese Gene können unter getrennter Regulation oder unter der gleichen Regulationsregion wie das erfindungsgemäße Desaturase-Gen liegen. Bei diesen Genen handelt es sich beispielsweise um weitere Biosynthesegene vorteilhaft der Fettsäure- und Lipidbiosynthese, die eine gesteigerte Synthese ermöglichen. Beispielsweise seien die Gene für die Δ15-, Δ12-, Δ9-, Δ6-, Δ5-Desaturase, die verschiedenen Hydroxylasen, die Acetylenase, die Acyl-ACP-Thioesterasen, die β-Ketoacyl-ACP-Synthasen, die Acyltransferasen wie die Diacylglycerolacyltransferase, die Glycerol-3-phosphatacyltransferase oder die Lysophosphatidsäureacyltransferase oder die β-Ketoacyl-ACP-Reductasen genannt. Vorteilhaft werden die Desaturasegene im Nukleinsäurekonstrukt verwendet bevorzugt das Δ12-Desaturasegen.

Das Nukleinsäurefragment wird zur Expression in einem Wirtsorganismus beispielsweise einem Mikroorganismus wie einem Pilz oder einer Pflanze vorteilhafterweise in einen Vektor wie beispielsweise einem Plasmid, einem Phagen oder sonstiger DNA inseriert, das eine optimale Expression der Gene im Wirt ermöglicht. Geeignete Plasmide sind beispielsweise in E. coli pLG338, pACYC184, pBR322, pUC18, pUC19, pKC30, pRep4, pHS1, pHS2, pPLc236, pMBL24, pLG200, pUR290, pIN-III¹¹³-B1, λgt11 oder pBdCI, in Streptomyces pIJ101, pIJ364, pIJ702 oder pIJ361, in Bacillus pUB110, pC194 oder pBD214, in Corynebacterium pSA77 oder pAJ667, in Pilzen pALS1, pIL2 oder pBB116, in Hefen 2µM, pAG-1, YEp6, YEp13 oder pEMBLYe23 oder in Pflanzen pLGV23, pGHlac⁺, pBIN19, pAK2004, pVKH oder pDH51 oder Derivate der vorstehend genannten Plasmide. Die genannten Plasmide stellen eine kleine Auswahl der möglichen Plasmide dar. Weitere Plasmide sind dem Fachmann wohl bekannt und können beispielsweise aus dem Buch Cloning Vectors (Eds. Pouwels P. H. et al. Elsevier, Amsterdam-New York-Oxford, 1985 , ISBN 0 444 904018) entnommen werden. Geeignete pflanzliche Vektoren werden unter anderem in "Methods in Plant Molecular Biology and Biotechnology" (CRC Press), Kap. 6/7, S.71-119 beschrieben.

Unter Vektoren sind außer Plasmiden auch alle anderen dem Fachmann bekannten Vektoren wie beispielsweise Phagen, Viren wie SV40, CMV, Baculovirus, Adenovirus, Transposons, IS-Elemente, Phasmide, Phagemide, Cosmide, lineare oder zirkuläre DNA zu verstehen. Diese Vektoren können autonom im Wirtsorganismus repliziert oder chromosomal repliziert werden. Bevorzugt ist eine chromosomale Replikation.

Der Vektor enthält vorteilhaft mindestens eine Kopie der erfindungsgemäßen Nukleinsäuresequenz und/oder des erfindungsgemäßen Nukleinsäurefragments.

Zur Erhöhung der Genkopienzahl können die Nukleinsäuresequenzen oder homologe Gene, beispielsweise in ein Nukleinsäurefragment bzw. in einen Vektor eingebaut werden, der vorzugsweise die den jeweiligen Genen zugeordnete, regulatorische Gensequenzen oder analog wirkende Promotoraktivität enthält. Insbesondere werden solche regulatorische Sequenzen verwendet, die die Genexpression verstärken.

Vorteilhafterweise enthält das Nukleinsäurefragment zur Expression der weiteren enthaltenen Gene zusätzlich noch 3' und/oder 5' Terminale regulatorische Sequenzen zur Steigerung der Expression, die je nach ausgewähltem Wirtsorganismus und Gen oder Gene für eine optimale Expression ausgewählt werden.

Diese regulatorischen Sequenzen sollen die gezielte Expression der Gene und der Proteinexpression ermöglichen. Dies kann beispielsweise je nach Wirtsorganismus bedeuten, daß das Gen erst nach Induktion exprimiert und/oder überexprimiert wird, oder daß es sofort exprimiert und/oder überexprimiert wird.

Die regulatorischen Sequenzen bzw. Faktoren können dabei vorzugsweise die Genexpression der eingeführten Gene positiv beeinflussen und dadurch erhöhen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird.

In einer weiteren Ausgestaltungsform des Vektors kann das erfindungsgemäße Genkonstrukt auch vorteilhafterweise in Form einer linearen DNA in die nicht-humanen Organismen eingeführt werden und über heterologe oder homologe Rekombination in das Genom des Wirtsorganismus integriert werden. Diese lineare DNA kann aus einem linearisierten Plasmid oder nur aus dem Nukleinsäurefragment als Vektor oder der erfindungsgemäßen Nukleinsäuresequenz bestehen.

Vorteilhafterweise wird die erfindungsgemäße Nukleinsäuresequenz zusammen mit mindestens einem Reportergen in ein Nukleinsäurekonstrukt kloniert, das in das Genom eingebracht wird. Dieses Reportergen sollte eine leichte Detektierbarkeit über einen Wachstums-, Fluoreszenz-, Chemo-, Biolumineszenz- oder Resistenzassay oder über eine photometrische Messung ermöglichen. Beispielhaft seien als Reportergene Antibiotika-oder Herbizidresistenzgene, Hydrolasegene, Fluoreszenzproteingene, Biolumineszenzgene, Zucker- oder Nukleotidstoffwechselgene oder Biosynthesegene wie das Ura3-Gen, das Ilv2-Gen, das Luciferasegen, das β-Galactosidasegen, das gfp-Gen, das 2-Desoxyglucose-6-phosphat-Phosphatasegen, das β-Glucuronidase-Gen, β-Lactamasegen, das Neomycinphosphotransferasegen, das Hygromycinphosphotransferasegen oder das BASTA (= Gluphosinat) Resistenz-Gen genannt. Diese Gene ermöglichen eine leichte Messbarkeit und Quantifizierbarkeit der Transkriptionsaktivität und damit der Expression der Gene. Damit lassen sich Genomstellen identifizieren, die eine unterschiedliche Produktivität zeigen.

In einer weiteren vorteilhaften Ausführungsform kann die erfindungsgemäße Nukleinsäuresequenz auch alleine in einen nicht-humanen Organismus eingebracht werden.

Sollen neben der erfindungsgemäßen Nukleinsäuresequenz weitere Gene in den Organismus eingeführt werden, so können alle zusammen mit einem Reportergen in einem einzigen Vektor oder jedes einzelne Gen mit einem Reportergen in je einem Vektor in den nicht-humanen Organismus eingebracht werden, wobei die verschiedenen Vektoren gleichzeitig oder sukzessive eingebracht werden können.

Der Wirtsorganismus enthält vorteilhaft mindestens eine Kopie der erfindungsgemäßen Nukleinsäure und/oder des erfindungsgemäßen Nukleinsäurekonstrukts.

Das Einbringen der erfindungsgemäßen Nukleinsäure, des Nukleinsäurekonstrukts oder des Vektors in nicht-humane Organismen beispielsweise Pflanzen kann prinzipiell nach allen dem Fachmann bekannten Methoden erfolgen.

Für Mikroorganismen kann der Fachmann entsprechende Methoden den Lehrbüchern von Sambrook, J. et al. (1989) Molecular cloning: A laboratory manual, Cold Spring Harbor Laboratory Press, von F.M. Ausubel et al. (1994) Current protocols in molecular biology, John Wiley and Sons, von D.M. Glover et al., DNA Cloning Vol.1, (1995), IRL Press (ISBN 019-963476-9), von Kaiser et al. (1994) Methods in Yeast Genetics, Cold Spring Habor Laboratory Press oder Guthrie et al. Guide to Yeast Genetics and Molecular Biology, Methods in Enzymology, 1994, Academic Press entnehmen.

Die Übertragung von Fremdgenen in das Genom einer Pflanze wird als Transformation bezeichnet. Es werden dabei die beschriebenen Methoden zur Transformation und Regeneration von Pflanzen aus Pflanzengeweben oder Pflanzenzellen zur transienten oder stabilen Transformation genutzt. Geeignete Methoden sind die Protoplastentransformation durch Polyethylenglykol-induzierte DNA-Aufnahme, die Verwendung einer Genkanone, die Elektroporation, die Inkubation trockener Embryonen in DNA-haltiger Lösung, die Mikroinjektion und der durch Agrobacterium vermittelte Gentransfer. Die genannten Verfahren sind beispielsweise in B. Jenes et al., Techniques for Gene Transfer, in: Transgenic Plants, Vol. 1, Engineering and Utilization, herausgegeben von S.D. Kung und R. Wu, Academic Press (1993) 128-143 sowie in Potrykus Annu. Rev. Plant Physiol.Plant Molec.Biol. 42 (1991) 205-225) beschrieben. Vorzugsweise wird das zu exprimierende Konstrukt in einen Vektor kloniert, der geeignet ist, Agrobacterium tumefaciens zu transformieren, beispielsweise pBin19 (Bevan et al., Nucl. Acids Res. 12 (1984) 8711). Die Transformation von Pflanzen mit Agrobacterium tumefaciens wird beispielsweise von Höfgen und Willmitzer in Nucl. Acid Res. (1988) 16, 9877 beschrieben.

Mit einem erfindungsgemäßen Expressionsvektor transformierte Agrobakterien können ebenfalls in bekannter Weise zur Transformation von Pflanzen wie Testpflanzen wie Arabidopsis oder Kulturpflanzen, insbesondere von Öl-haltigen Kulturpflanzen, wie Soja, Erdnuß, Rizinus, Sonnenblume, Mais, Baumwolle, Flachs, Raps, Kokosnuß, Ölpalme, Färbersaflor (Carthamus tinctorius) oder Kakaobohne verwendet werden, z.B. indem verwundete Blätter oder Blattstücke in einer Agrobakterienlösung gebadet und anschließend in geeigneten Medien kultiviert werden.

Die genetisch veränderten Pflanzenzellen können über alle dem Fachmann bekannten Methoden regeneriert werden. Entsprechende Methoden können den oben genannten Schriften von S.D. Kung und R. Wu, Potrykus oder Höfgen und Willmitzer entnommen werden.

Als Organismen bzw. Wirtsorganismen für die erfindungsgemäße Nukleinsäure, das Nukleinsäurekonstrukt oder den Vektor eignen sich prinzipiel alle nicht-humanen Organismen, die in der Lage sind Fettsäuren speziell ungesättigte Fettsäuren zu synthetisieren bzw. für die Expression rekombinanter Gene geeignet sind. Beispielhaft seien Pflanzen wie Arabidopsis, Asteraceae wie Calendula oder Kulturpflanzen wie Soja, Erdnuß, Rizinus, Sonnenblume, Mais, Baumwolle, Flachs, Raps, Kokosnuß, Ölpalme, Färbersaflor (Carthamus tinctorius) oder Kakaobohne, Mikroorganismen wie Pilze beispielsweise die Gattung Mortierella, Saprolegnia oder Pythium, Bakterien wie die Gattung Escherichia, Hefen wie die Gattung Saccharomyces, Algen oder Protozoen wie Dinoflagellaten wie Crypthecodinium genannt. Bevorzugt werden Organismen, die natürlicherweise Öle in größeren Mengen synthetisieren können wie Pilze wie Mortierella alpina, Pythium insidiosum oder Pflanzen wie Soja, Raps, Flachs, Kokosnuß, Ölpalme, Färbersaflor, Rizinus, Calendula, Erdnuß, Kakaobohne oder Sonnenblume oder Hefen wie Saccharomyces cerevisiae, besonders bevorzugt werden Soja, Raps, Flachs, Sonnenblume, Calendula oder Saccharomyces cerevisiae. Prinzipiell sind als Wirtsorganismen auch nicht-humane transgene Tiere beispielsweise Caenorhabditis elegans.

Eine weitere erfindungsgemäße Ausgestaltung sind wie oben beschrieben transgene Pflanzen, die eine funktionelle oder nicht funktionelle Nukleinsäure oder ein funktionelles oder nicht funktionelles Nukleinsäurekonstrukt enthalten. Unter nicht funktionell ist zu verstehen, daß kein enzymatisch aktives Protein mehr synthetisiert wird, da das natürliche Gen inaktiviert wurde. Außerdem ist unter nicht funktionellen Nukleinsäuren oder Nukleinsäurekonstrukten auch eine sogenannte Antisense-DNA zu verstehen, die zu transgenen Pflanzen führt, die eine Reduktion der enzymatischen Aktivität oder keine enzymatischen Aktivität aufweisen. Mit Hilfe der Antisense-Technik, speziell wenn die erfindunsgemäße Nukleinsäuresequenz mit anderen Fettsäuresynthesegene in der Antisense-DNA kombiniert wird, ist es möglich Triglyceride mit einem erhöhten Gehalt an gesättigten Fettsäuren bzw. gesättigte Fettsäuren zu synthetisieren. Unter transgenen Pflanzen sind einzelne Pflanzenzellen und deren Kulturen auf Festmedien oder in Flüssigkultur, Pflanzenteile und ganze Pflanzen zu verstehen.

Die Verwendung der erfindungsgemäßen Nukleinsäuresequenz oder des erfindungsgemäßen Nukleinsäurekonstruktes zur Herstellung von transgenen Pflanzen gehört deshalb auch zu den Erfindungsgegenständen.

Ein weiterer Gegenstand der Erfindung ist ein Enzym, das eine Fettsäure der allgemeinen Struktur I, 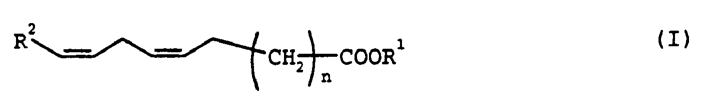 die zwei durch eine Methylengruppe voneinander getrennte Doppelbindungen aufweist, zu einer dreifach ungesättigten Fettsäure der allgemeinen Struktur II 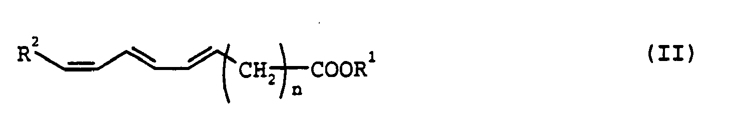 umsetzt, wobei die drei Doppelbindungen der Fettsäure in Konjugation sind und wobei die Substituenten und Variablen in den Verbindungen der allgemeinen Struktur I und II folgende Bedeutung haben:
- R¹: = Wasserstoff, substituiertes oder unsubstituiertes, ungesättigte oder gesättigtes, verzweigtes oder unverzweigtes C₁-C₁₀-Alkyl-, 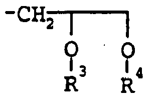
- R²: = substituiertes oder unsubstituiertes, ungesättigtes oder gesättigtes C₁-C₉-Alkyl-
- R³: und R⁴ unabhängig voneinander Wasserstoff, substituiertes oder unsubstituiertes, gesättigtes oder ungesättigtes, verzweigtes oder unverzweigtes C₁-C₂₂-Alkylcarbonyl- oder Phospho-,
- n: = 1 bis 14, bevorzugt 1 bis 8, besonders bevorzug 4 bis 6, ganz besonders bevorzugt 6.

R¹ bezeichnet in den Verbindungen der Formeln I und II Wasserstoff, substituiertes oder unsubstituiertes, ungesättigte oder gesättigtes, verzweigtes oder unverzweigtes C₁-C₁₀-Alkyl-, oder

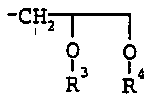

Als Alkylreste seien substituierte oder unsubstituierte verzweigte oder unverzweigte C₁-C₁₀-Alkylketten wie beispielsweise Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl, n-Nonyl oder n-Decyl genannt.

Bevorzugte Reste für R¹ sind Wasserstoff und

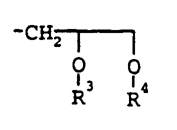

R² bezeichnet in den Verbindungen der Formeln I und II substituiertes oder unsubstituiertes, ungesättigtes oder gesättigtes C₁-C₉-Alkyl-.

Als Alkylreste seien substituierte oder unsubstituierte verzweigte oder unverzweigte C₁-C₉-Alkylketten wie beispielsweise Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl oder n-Nonyl genannt. Bevorzugt ist C₁-C₅-Alkyl, besonders bevorzugt ist C₅-Alkyl.

R³ und R⁴ bezeichnen unabhängig voneinander Wasserstoff, substituiertes oder unsubstituiertes, gesättigtes oder ungesättigtes, verzweigtes oder unverzweigtes C₁-C₂₂-Alkylcarbonyl- oder Phospho-.

C₁-C₂₂-Alkylcarbonyl wie Methylcarbonyl, Ethylcarbonyl, n Propylcarbonyl, 1 Methylethyl carbonyl, n Butylcarbonyl, 1 Methylpropylcarbonyl, 2 Methylpropylcarbonyl, 1,1 Dimethylethylcarbonyl, n Pentylcarbonyl, 1 Methylbutylcarbonyl, 2 Methylbutylcarbonyl, 3 Methylbutylcarbonyl, 1,1 Dimethylpropylcarbonyl, 1,2 Dimethylpropylcarbonyl, 2,2 Dimethylpropylcarbonyl, 1 Ethylpropylcarbonyl, n Hexylcarbonyl, 1 Methylpentylcarbonyl, 2 Methylpentylcarbonyl, 3 Methylpentylcarbonyl, 4 Methylpentylcarbonyl, 1,1 Dimethylbutylcarbonyl, 1,2 Dimethylbutylcarbonyl, 1,3 Dimethylbutylcarbonyl, 2,2 Dimethylbutylcarbonyl, 2,3 Dimethylbutylcarbonyl, 3,3 Dimethylbutylcarbonyl, 1 Ethylbutylcarbonyl, 2 Ethylbutylcarbonyl, 1,1,2 Trimethylpropylcarbonyl, 1,2,2 Trimethylpropylcarbonyl, 1 Ethyl 1 methylpropylcarbonyl und 1 Ethyl 2 methylpropylcarbonyl, Heptylcarbonyl, Nonylcarbonyl, Decylcarbonyl, Undecylcarbonyl, n-Dodecylcarbonyl, n-Tridecylcarbonyl, n-Tetradecylcarbonyl, n-Pentadecylcarbonyl, n-Hexadecylcarbonyl, n-Heptadecylcarbonyl, n-Octadecylcarbonyl, n-Nonadecylcarbonyl oder n-Eicosylcarbonyl.

Bevorzugte Substituenten für R³ und R⁴ sind gesättigtes oder ungesättigtes C₁₆-C₂₂-Alkylcarbonyl.

Als Substituenten der genannten Reste seien beispielsweise Halogen wie Fluor oder Chlor, Alkyl oder Hydroxyl genannt.

Bei der Umsetzung mit dem erfindungsgemäßen Enzym wird eine Doppelbindung in die Fettsäure eingeführt und eine Doppelbindung verschoben, so daß die an der Reaktion beteiligten drei Doppelbindungen in Konjugation liegen. Weiterhin wird eine Doppelbindung isomerisiert (von cis zu trans).

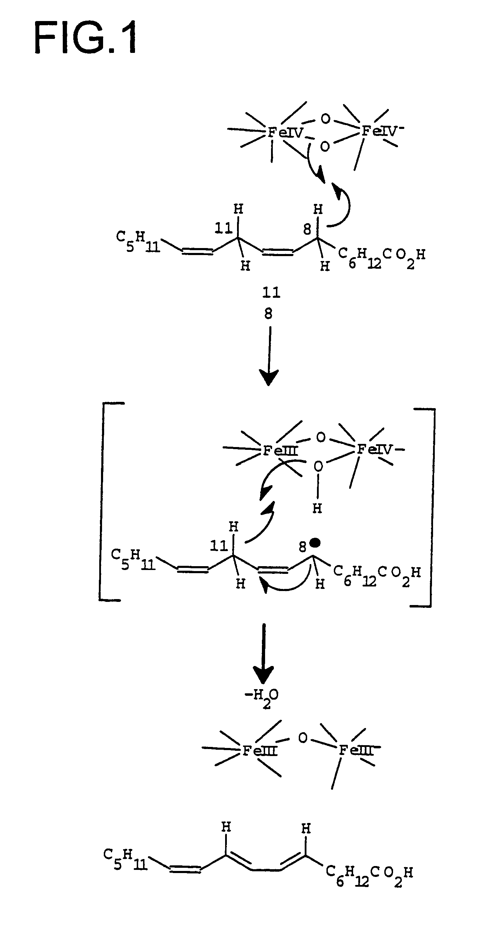

Das Enzym (= Calendulasäure-Desaturase) katalysiert vorteilhaft die Umsetzung von Linolsäure (18:2, 9Z,12Z) zu Calendulasäure (18:3, 8E,10E,12Z). Das Enzym führt eine trans-Doppelbindung an Position C8 ein und bewirkt die spezifische Verschiebung einer cis-Doppelbindung in Position C9 zu einer trans-Doppelbindung in Position C10, wobei die Isomerisierung regiospezifisch erfolgt. Ein möglicher hypothetischer Reaktionsmechanismus ist in Fig. 1 dargestellt. Nach einer Deprotonierung an C8 der Linolsäure und einer Umlagerung des Radikals nach C10 kommt es im Zuge einer Wasserabspaltung zur Deprotonierung an C11 und damit zur Bildung von Calendulasäure. Gleichzeitig wird gebundenes Fe IV zu Fe III reduziert. Fig. 1 gibt den hypothetischen Mechanismus für (8,11)-Linoleoyl Desaturase (Calendulasäure-Desaturase) modifiziert nach Svatos, A et al. (Insect Biochemistry and Molecular Biology 29,1999:225-232) basierend auf dem vorgeschlagenen Katalysemechanismus für Δ9 Desaturase aus Ricinus (Lindqvist, Y et al., EMBO Journal 15, 1996:4081-4092) wieder. Als Substrate kommen weiterhin auch die 6Z,9Z,12Z, 18:3-Fettsäure und die 9Z,12Z,15Z, 18:3-Fettsäure in Frage, die dann zu 6Z,8E,10E,12Z-bzw. 8E,10E,12Z,15Z-Fettsäuren umgesetzt werden.

Ein weiterer erfindungsgemäßer Gegenstand ist ein Verfahren zur Herstellung von ungesättigten Fettsäuren dadurch gekennzeichnet, daß man mindestens eine oben beschriebene erfindungsgemäße Nukleinsäuresequenz oder mindestens ein erfindungsgemäßes Nukleinsäurekonstrukt in einen bevorzugt Öl produzierenden nicht-humanen Organismus bringt, diesen Organismus anzieht und daß in dem Organismus enthaltene Öl isoliert und die im Öl enthaltenden Fettsäuren freisetzt.

Als Organismen für die genannten Verfahren seien beispielhaft Pflanzen wie Arabidopsis, Soja, Erdnuß, Rizinus, Sonnenblume, Mais, Baumwolle, Flachs, Raps, Kokosnuß, Ölpalme, Färbersaflor (Carthamus tinctorius) oder Kakaobohne, Mikroorganismen wie Pilze Mortierella, Saprolegnia oder Pythium, Bakterien wie die Gattung Escherichia, Hefen wie die Gattung Saccharomyces, Algen oder Protozoen wie Dinoflagellaten wie Crypthecodinium genannt. Bevorzugt werden Organismen, die natürlicherweise Öle in größeren Mengen synthetisieren können wie Pilze wie Mortierella alpina, Pythium insidiosum oder Pflanzen wie Soja, Raps, Flachs, Kokosnuß, Ölpalme, Färbersaflor, Rizinus, Calendula, Erdnuß, Kakaobohne oder Sonnenblume oder Hefen wie Saccharomyces cerevisiae, besonders bevorzugt werden Soja, Raps, Flachs, Sonnenblume, Calendula oder Saccharomyces cerevisiae.

Die in den Verfahren verwendeten Organismen werden je nach Wirtsorganismus in dem Fachmann bekannter Weise angezogen bzw. gezüchtet. Mikroorganismen werden in der Regel in einem flüssigen Medium, das eine Kohlenstoffquelle meist in Form von Zuckern, eine Stickstoffquelle meist in Form von organischen Stickstoffquellen wie Hefeextrakt oder Salzen wie Ammoniumsulfat, eine Phosphatquelle wie Kaliumhydrogenphosphat, Spurenelemente wie Eisen-, Mangan-, Magnesiumsalze und gegebenenfalls Vitamine enthält, bei Temperaturen zwischen 0 °C und 100 °C, bevorzugt zwischen 10 °C bis 60 °C unter Sauerstoffbegasung angezogen. Dabei kann der pH der Nährflüssigkeit auf einen festen Wert gehalten werden, das heißt während der Anzucht wird der pH reguliert. Es ist auch eine Anzucht ohne pH-Regulation möglich. Die Anzucht kann im batch, semi batch oder kontinuierlich erfolgen. Nährstoffe können zu Beginn der Fermentation vorgelegt oder semikontinuierlich oder kontinuierlich nach gefüttert werden.

Pflanzen werden nach Transformation zunächst wie oben beschrieben regeneriert und anschließend wie üblich angezüchtet bzw. angebaut.

Aus den nicht-humanen Organismen werden nach Anzucht die Lipide in üblicherweise gewonnen. Hierzu können die nicht-humanen Organismen nach Ernte zunächst aufgeschlossen werden oder direkt verwendet werden. Die Lipide werden vorteilhaft mit geeigneten Lösungsmitteln wie apolare Lösungsmittel wie Hexan oder polaren wie Ethanol, Isopropanol oder Gemischen wie Hexan/Isopropanol, Phenol/Chloroform/Isoamylalkohol bei Temperaturen zwischen 0 °C bis 80 °C, bevorzugt zwischen 20 °C bis 50 °C extrahiert. Die Biomasse wird in der Regel mit einem Überschuß an Lösungsmittel extrahiert beispielsweise einem Überschuß von Lösungmittel zu Biomasse von 1:4. Das Lösungmittel wird anschließend beispielsweise über eine Destillation entfernt. Die Extraktion kann auch mit superkritischem CO₂ erfolgen. Nach Extraktion kann die restliche Biomasse beispielsweise über Filtration entfernt werden. Standardmethoden zur Extraktion von Fettsäuren aus Pflanzen und Mikroorganismen werden in Bligh et al. (Can. J. Biochem. Physiol. 37, 1959: 911-917) oder Vick et al. (Plant Physiol. 69, 1982: 1103-1108) beschrieben.

Das so gewonnene Rohöl kann anschließend weiter aufgereinigt werden, beispielsweise in dem Trübungen über das Versetzen mit polaren Lösungsmitteln wie Aceton oder apolaren Lösungsmitteln wie Chloroform und anschließender Filtration oder Zentrifugation entfernt werden. Auch eine weitere Reinigung über Säulen oder anderen Techniken ist möglich.

Zur Gewinnung der freien Fettsäuren aus den Triglyceriden werden diese in üblicherweise verseift beispielsweise mit NaOH oder KOH.

Ein weiterer Gegenstand der Erfindung sind ungesättigte oder gesättigte Fettsäuren sowie Trigylceride mit einem erhöhten Gehalt and gesättigten oder ungesättigten Fettsäuren, die nach den oben genannten Verfahren hergestellt wurden sowie deren Verwendung zur Herstellung von Nahrungsmitteln, Tierfutter, Kosmetika oder Pharmazeutika. Hierzu werden diese den Nahrungsmitteln, dem Tierfutter, den Kosmetika oder Pharmazeutika in üblichen Mengen zugesetzt.

Die Erfindung wird in den folgenden Beispielen näher erläutert:

### Beispiele

Über RT-PCR und RACE-Techniken wurde aus mRNA von Calendula officinalis eine cDNA kloniert. Bei Expression dieser cDNA in Hefe wird Linolsäure in das Octadecakonjutrien Calendulasäure (8E, 10E, 12Z) umgesetzt. Es handelt sich hierbei unseres Wissens nach um die erstmalige Beschreibung einer Calendulasäure-Desaturase. Das Enzym bewirkt eine regiospezifische Verschiebung einer *cis*-Doppelbindung in Position C9 zu einer *trans*-Doppelbindung in Position C10 und führt eine neue trans-Doppelbindung an Position C8 ein.

Transgene Hefen und Pflanzen mit erhöhter Expression der Calendulasäure-Desaturase-cDNA weisen Calendulasäure in ihren Lipiden auf.

### Beispiel 1: RNA-Isolierung aus Samen von Calendula officinalis

Um cDNA-Klone für Calendulasäure-Desaturase per PCR isolieren zu können, wurde RNA aus Samen von Calendula officinalis präpariert. Aufgrund des hohen Fettgehalts der Samen konnten hierzu keine Standardprotokolle verwendet werden, sondern es wurde die folgende Methode angewandt:

20 g Pflanzenmaterial wurden in flüssigem Stickstoff zu einem Pulver zermörsert. 100 ml Extraktionspuffer 1 [100 mM Tris/HCl, pH 7,5, 25 mM EDTA, 2 % (w/v) Laurylsarkosyl, 4 M Guanidiniumthiocyanat, 5 % (w/v) PVP (= Polyvinylpyrrolidon), 1% (v/v) β-Mercaptoethanol] wurden zugegeben, sofort durchmischt und homogenisiert. Die Lösung wurde in 50 ml-Gefäße überführt und für ca. 15 min geschüttelt. Nach einer Zentrifugation bei 4000 g für 10-15 min wurde die oben schwimmende Fettschicht bzw. Fettropfen abgenommen und der Überstand in frische Gefäße überführt. Es folgte eine Extraktion mit 1 Volumen Phenol/Chloroform/Isoamylalkohol (= PCI, 25:24:1) und eine Extraktion mit Chloroform, wobei jeweils 15 Minuten lang geschüttelt und dann abzentrifugiert wurde. Die obere, wässrige Phase wurde abgenommen, auf ein 8 ml CsCl-Kissen (5 M CsCl) geschichtet und 18 Stunden lang bei 18 °C und 100 000 g zentrifugiert. Der Überstand wurde dekantiert und das RNA-Präzipitat kurz getrocknet. Nach einem Waschschritt mit 70 % Ethanol wurde die RNA in einer Mischung aus 7,5 ml Extraktionspuffer II (100 ml Tris/HCl, pH 8,8, 100 mM NaCl, 5 mM EDTA, 2 % SDS) und 10 ml PCI gelöst, 15 Minuten lang geschüttelt und abzentrifugiert. Die obere, wässrige Phase wurde nach einer Chloroform-Extraktion mit dem gleichen Volumen 5 M LiCl versetzt. Die RNA-Fällung erfolgte über Nacht bei 4 °C. Anschließend wurde 60 Minuten lang bei 12000 g und 4 °C abzentrifugiert. Das Präzipitat wurde zwei mal mit 70 % Ethanol gewaschen und getrocknet und schießlich in 500 µl H₂O aufgenommen.

Aus der so gewonnen Calendula-Gesamt-RNA wurde mit dem Poly-Attract-Kit (Promega, Mannheim) nach Angaben des Herstellers mRNA isoliert. 1 µg dieser mRNA wurden mit der SuperscriptII reversen Transkriptase von Gibco BRL (Eggenstein) mit 200 pmol oligo-dT-Primer nach Herstellervorschrift in cDNA übersetzt und als Template in einer Polymerasekettenreaktion (PCR) eingesetzt.

### Beispiel 2 : Isolierung und Klonierung der Calendulasäure-Desaturase aus Calendula officinalis

Um DNA-Sequenzen aus Calendula officinalis zu isolieren, die für eine Calendulasäure-Desaturase kodieren, wurden verschiedene degenerierte Oligonukleotidprimer von Aminosäure-Sequenzen der konservierten Histidin-Boxen verschiedener Δ12-Desaturasen abgeleitet.
Primer A: 5' - CCD TAY TTC TCI TGG AAR WWH AGY CAY CG - 3'
Forward primer, abgeleitet von der Aminosäuresequenz P Y F S W K Y/I S H R
Primer B: 5' - CCA RTY CCA YTC IGW BGA RTC RTA RTG - 3'
Reverse primer, abgeleitet von der Aminosäuresequenz H Y D S S/T E W D/N W

Die Buchstaben in Primer A und B haben folgende Bedeutung:
R = A/G
Y = C/T
W = A/T
H = A/C/T
B = C/G/T
D = A/G/T
I = Inositol

In einer PCR mit Calendula-Einzelstrang-cDNA (hergestellt nach Beispiel 1) als Template wurde mit den Primern A und B ein DNA-Fragment mit einer Länge von 470 bp amplifiziert. Es wurde folgendes PCR-Programm verwendet:

| | | |
|---|---|---|
| 1. | 2 min | 94 °C |
| 2. | 30 sec | 94 °C |
| 3. | 45 sec | 50 °C (Bindungstemperatur) |
| 4. | 1 min | 72 °C |
| | 10 x 2. bis 4. | |
| 5. | 0 sec | 94 °C |
| 6. | 45 sec | 50 °C |
| 7. | 1 min | 72 °C, Zeitincrement 5 sec pro Zyklus |
| | 20 x 5. Bis | 7. |
| 8. | 2 min | 72 °C |

Für die Amplifikation wurde die TfI-DNA-Polymerase von Biozym (Hess. Oldendorf) verwendet. Das 470 bp lange DNA-Fragment wurde mit Hilfe des TOPO TA Cloning Kits (Invitrogen, Carlsbad, USA) in den Vektor pCR 2.1-TOPO kloniert und sequenziert. Die Sequenz des 470 bp-Fragments entsprach der Sequenz von Nukleotid 466 bis 893 von SEQ ID NO:1.

### Beispiel 3: Gewinnung und Sequenzierung vollständiger cDNA-Klone

Um einen Vollängen-Klon zu erhalten, wurde das Fragment mittels 5'- und 3'- RACE (rapid amplification of cDNA ends) verlängert. Ausgehend von 1 µg mRNA (isoliert nach Beispiel 1) wurde mit dem "Marathon cDNA Amplification Kit" von CLONTECH (Heidelberg) doppelsträngige cDNA hergestellt. Nach erfolgter Adaptorligation wurde mit folgenden Primern 5'- bzw. 3'-RACE durchgeführt:

### Spezifische Primer für 5'-RACE:

Primer C 5' - GTG AGG GAG TGA GAG ATG GGT GTG GTG C - 3'
Primer D 5' - AAC ACA CTT ACA CCT AGT ACT GGA ATT G - 3'

### Spezifische Primer für 3'-RACE:

Primer E 5' - TAT TCC AAA CTT CTT AAC AAT CCA CCC G - 3'
Primer F 5'- CAA TTC CAG TAC TAG GTG TAA GTG TGT T - 3'

Zunächst wurde eine PCR mit der adaptorligierten doppelsträngigen -cDNA und Primer C bzw. E durchgeführt, danach erfolgte eine zweite PCR mit Primer D bzw. F und einer 1:50-Verdünnung des PCR-Produkts aus der Reaktion mit Primer C bzw. E als Template.

Die RACE-PCR erfolgte nach folgendem Programm:

| | | |
|---|---|---|
| 1. | 1 min | 94 °C |
| 2. | 30 sec | 94 °C |
| 3. | 3 min | 68 °C |
| | 10 x 2. - 3. | |
| 4. | 30 sec | 94 °C |
| 5. | 30 sec | 65 °C |
| 6. | 3 min | 68 °C |
| | 25 x 4. - 6. | |
| 7. | 5 min | 68 °C |

Die erhaltenen DNA-Fragmente wurden mit Hilfe des TOPO TA Cloning-Kits (Invitrogen, Carlsbad, USA) in pCR 2.1-TOPO kloniert und sequenziert. Das 5'-RACE-Produkt reichte über das Startkodon in den 5'-nicht-translatierten-Bereich (5'-UTR), das 3'-RACE über das Stopkodon in den 3'- UTR hinein.

Die zusammengesetzte Sequenz bestehend aus dem ersten PCR-Produkt und den RACE-Produkten ist in SEQ ID NO: 1 dargestellt. Der kodierende Bereich erstreckt sich von Nukleotid 42 (Startkodon) bis 1175 (Stopkodon). Die 5'- und 3'-UTRs wurden nur einzelsträngig sequenziert, so daß hier einzelne Sequenzierfehler möglich sind.

Um einen durchgängigen Vollängen-Klon zu erhalten, wurde mit dem Expand High Fidelity-System (Boehringer, Mannheim) und den Primern G und H sowie mit Calendula-cDNA (siehe Beispiel 1) als Template eine PCR durchgeführt.
Primer G 5' - ATTA**GAGCTC**ATGGGTGCTGGTGGTCGGATGTCG - 3' Forward Primer (mit **SacI**-Schnittstelle)
Primer H 5' - ATTACT**CGAGTG**ACATACACCTTTTTGATTACATCTTG - 3' Reverse Primer (mit **XhoI**-Schnittstelle)

Die PCR erfolgte nach folgendem Programm:

| | | |
|---|---|---|
| 1. | 2 min | 94 °C |
| 2. | 30 sec | 94 °C |
| 3. | 35 sec | 63 °C |
| 4. | 2 min | 72 °C |
| | 10x2.-4. | |
| 5. | 30 sec | 94 °C |
| 6. | 35 sec | 63 °C |
| 7. | 2 min | 72 °C, Zeitincrement 5 sec pro Zyklus |
| | 15 x 5. - 7 | |
| 8. | 2 min | 72 °C. |

Das PCR-Produkt mit einer Länge von 1,2 kb wurde in den Vektor pGEM-T (Promega, Mannheim) kloniert und in E. coli DH10B transformiert. Die Insert-DNA wurde mit einem 373 DNA-Sequencer (Applied Biosystems) doppelsträngig sequenziert. Dazu wurden neben Reverse Primer und -21 Primer folgende sequenzspezifische Primer benutzt:
Primer I: 5' - CGG TCT TCT CGC TGT ATT - 3'
Primer J: 5' - ATT ACC CAA GCT GCC C - 3'

Die vollständige DNA-Sequenz der Calendulasäure-Desaturase (CalDes) ist identisch mit dem Abschnitt von Nukleotid 42 bis 1193 von SEQ ID NO:1. Die Sequenz umfaßt den kodierenden Bereich und einen kurzen Abschnitt des 3'-UTR.

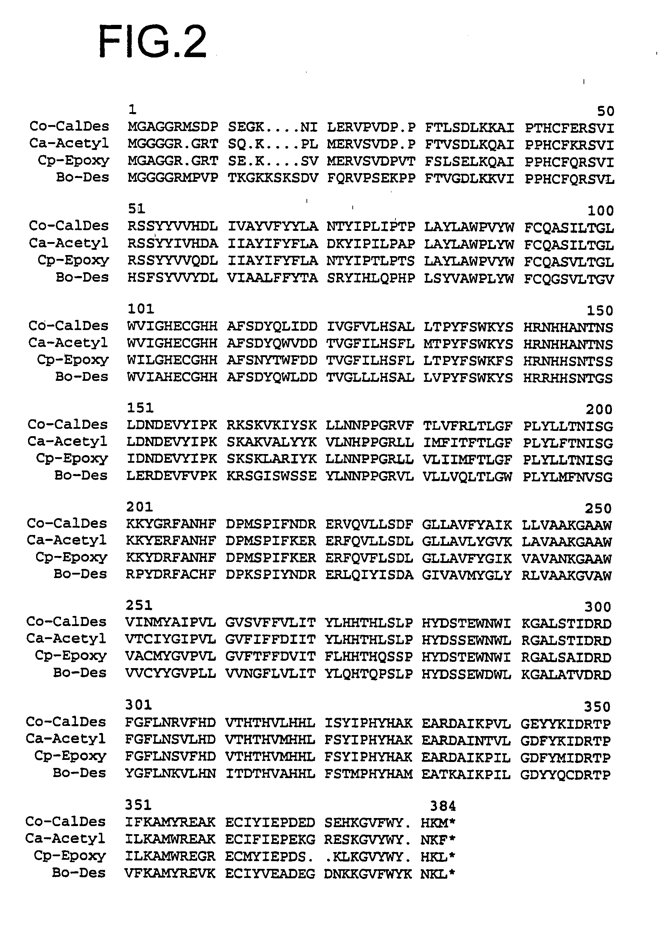

Ein Vergleich der abgeleiteten Aminosäuresequenz von Co-CalDes (SEQ ID NO:2) mit annotierten Proteinsequenzen der SWISS-PROT und SP-TREMBL-Datenbanken ergab die höchste Homologie zu einer Δ12-Acetylenase aus Crepis alpina (SP_PL: 081931, 74 % identische Aminosäuren), einer Δ12-Epoxygenase aus Crepis palaestina (SP_PL: 065771, 73 % identische Aminosäuren) und einer Δ12-Desaturase aus Borago officinalis (SP_PL: 082729, 62 % identische Aminosäuren) über den gesamten kodierenden Bereich. Die Sequenzvergleiche sind in Fig.2 dargestellt. Fig. 2 zeigt einen Vergleich der Aminosäure-Sequenzen von Co-CalDes mit Δ12-Acetylenase aus Crepis alpina (Ca-Acetyl), Δ12-Epoxygenase aus Crepis palaestina (Cp-Epoxy) und Δ12-Desaturase aus Borago officinalis (Bo-Des).

### Beispiel 4: Expression der Calendulasäure-Desaturase in Hefe

Um die Funktionalität von CalDes nachzuweisen, wurde in einem ersten Ansatz der kodierende Bereich der cDNA in einem Hefe-Expressionsvektor kloniert und in S. cerevisiae exprimiert. Die in der Hefe produzierte Calendulasäure-Desaturase sollte zugesetzte Linolsäure in Calendulasäure umsetzen. Diese wiederum sollte in hydrolisierten Lipidextrakten über HPLC nachgewiesen werden.

In einem zweiten Ansatz wurde zusätzlich zu CalDes die Δ12-Desaturase FAD2 aus A. thaliana (Kajiwara et al., Appl. Environ. Microbiol., 62, 1996: 4309 - 4313) in Hefe exprimiert, so daß die Hefezellen endogen Linolsäure produzieren, die dann wiederum durch die Aktivitität von CalDes in Calendulasäure umgesetzt werden kann. Letztere sollte wiederum über HPLC nachgewiesen werden.

Sämtliche Fest- und Flüssigmedien für Hefe wurden nach Protokollen von Ausubel et al. (Current Protocols in Molecular Biology, John Wiley & Sons, New York, 1995) hergestellt.

Die CalDes-cDNA wurde über Restriktionsverdau mit SacI/XhoI aus dem Vektor pGEM-T ausgeschnitten, in den SacI/XhoI geschnittenen shuttle-Vektor pYES2 (Invitrogen, Carlsbad, USA) kloniert und der so entstandene Vektor pYES2-CalDes in E. coli XL1 blue tränsformiert. Nach erneuter Plasmidpräparation mit Hilfe des Plasmid Maxi Kits (QIAGEN) wurde pYES2-CalDes mit Hilfe der Polyethylenglycol-Methode (Von Pein M., Dissertation, Heinrich Heine-Universität Düsseldorf, 1992) in S. cerevisiae INCSv1 (Invitrogen, Carlsbad, USA) transformiert, wo die Expression der CalDes-cDNA unter der Kontrolle des GAL1-Promotors stand.

Um im zweiten Ansatz zusätzlich zu CalDes auch FAD2 in Hefe exprimieren zu können, wurde zunächst der kodierende Bereich des FAD2-Gens über PCR (Protokoll siehe Primer G und H) aus A. thaliana-cDNA mit Hilfe der Tfl-Polymerase (Biozym) amplifiziert. Folgende Primer wurden hierfür verwendet:
Primer K: 5' - AAA**CTCGAG**ATGGGTGCAGGTGGAAGAATGCCGG - 3' Forward Primer (**XhoI**-Schnittstelle)
Primer L: 5' - AAA**AAGCTT**TCATAACTTATTGTTGTACCAGTACACACC - 3' Reverse Primer (**HindIII**-Schnittstelle)

Das entstandene PCR-Produkt wurde nach Restriktionsverdau mit XhoI/HindIII in den XhoI/HindIII geschnittenen Hefe-Expressionsvektor pESC-Leu (Stratagene) kloniert, wo die FAD2-DNA unter Kontrolle des GAL1-Promotors stand.

Die Expression von CalDes in S. cerevisiae INCSv1 erfolgte modifiziert nach Avery et al. (Appl. Environ. Microbiol., 62, 1996: 3960 - 3966) und Girke et al. (The Plant Journal, 5, 1998: 39 - 48). Um eine Starterkultur herzustellen, wurden 10 ml YPAD-Medium mit einer Einzelkolonie angeimpft und 48 Stunden lang bei 30 °C bei 200 rpm inkubiert. Die Zellkultur wurde dann in 1 x YPA-Medium ohne Zucker gewaschen und abzentrifugiert. Die pelletierten Zellen wurden in 2 ml Minimalmedium ohne Supplemente und ohne Zucker resuspendiert. Mit 1 ml dieser Zellsuspension wurden 100 ml Minimalmedium (dropout powder, 2 % Raffinose, 1 % Tergitol NP40) in 500 ml Erlenmeyekolben angeimpft und die Kultur bei 30 °C und 200 rpm kultiviert. Bei einer OD₆₀₀ von 0,5 wurden 2 % (w/v) Galaktose zugegeben und (für den ersten Ansatz) 0,003 % Linolsäure (3%ige Stammlösung in 5 % Tergitol NP40). Die Zellen wurden weiter kultiviert bis zum Erreichen der stationären Phase. Dann wurden sie in Minimalmedium ohne Supplemente gewaschen und bei -20 °C aufbewahrt.

### Beispiel 5: Lipidextraktion und HPLC-Analyse der Fettsäuren aus transgener Hefe

Die Hefezellen wurden in 30 ml HIP-Lösung (0,1 mM 2,6-Di-tert.-butyl-4-methyl-phenol in Hexan : Isopropanol (3:2 v/v)) suspendiert, mit 150 µl konzentrierter HCl angesäuert und mit Ultra-Turrax homogenisiert (1 min, 24000 rpm). Anschließend wurden die Proben 10 min lang bei 4 °C geschüttelt und bei 5000 g und 4 °C 10 min lang abzentrifugiert. Der Überstand wurde in ein neues Gefäß überführt und mit 0,38 M K₂SO₄ auf 47,5 ml aufgefüllt. Die Proben wurden wiederum 10 min lang bei 4 °C geschüttelt und abzentrifugiert (s.o.). Die Hexanphase wurde abgenommen und unter N₂-Strom eingedampft. Der Rückstand wurde in 20 µl Chloroform gelöst. Zur alkalischen Hydrolyse von Fettsäure-Estern wurden 400 µl Methanol sowie 80 µl 40 %ige (w/v) KOH-Lösung zugegeben und die Probe 20 min lang bei 60 °C unter Argon inkubiert. Anschließend wurde die Probe auf Raumtemperatur abgekühlt, mit 35 µl konzentrierter HCl auf pH 3,0 angesäuert und per HPLC aufgetrennt.

Die Trennung der freien Fettsäuren erfolgte mit einer ET 250/4 Nucleosil 120-5 C18-Säule (Macherey & Nagel). Als Laufmittel diente Methanol: H₂O: Eisessig (85:15:0,1 v/v/v). Die Trennung erfolgte bei einer Flußrate von 1 ml/min und 25 °C, zur Detektion der Konjutriene wurde die Absorption bei 268 nm gemessen.

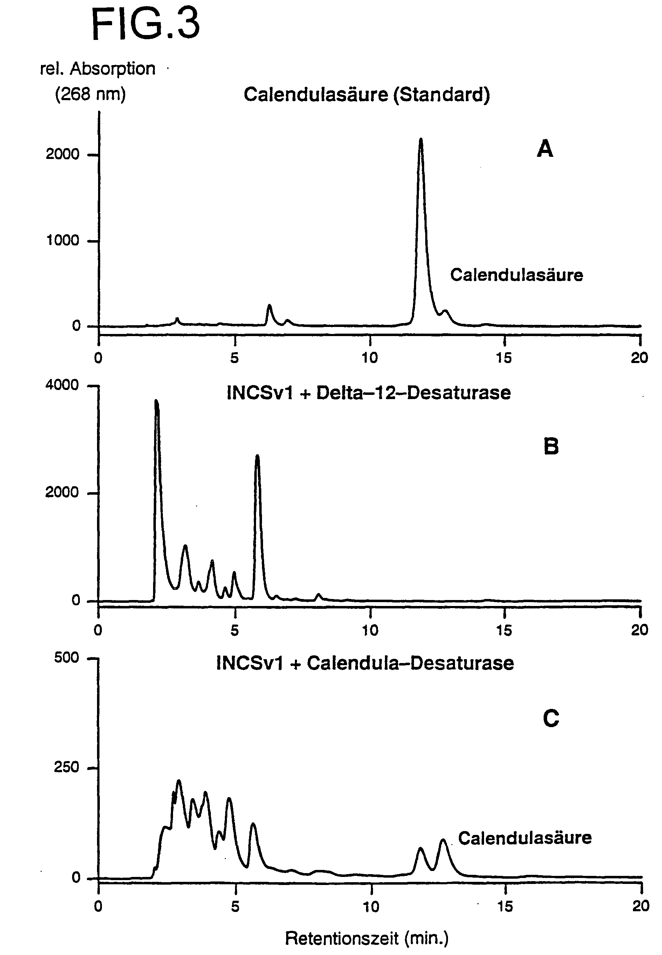

Fig. 3 zeigt die Elutionsprofile der Lipidextrakte nach alkalischer Hydrolyse aus transformierten Hefezellen (Fig. 3B, Elutionsprofil von S. cerevisiae INCSv1 transformiert mit FAD2-DNA aus A. thaliana und C, Elutionsprofil von *S. cerevisiae* INCSv1 transformiert mit pYES2-CalDes aus Calendula officinalis) bzw. das Elutionsprofil eines Calendulasäure-Standards (Fig. 3A). Calendulasäure hat eine Retensionszeit von 12 min mit einer für Konjutriene typischen, starken Absorption bei 268 nm. Die hydrolisierten Lipidextrakte von Hefezellen, die mit dem leeren Vektor pYES2 transformiert und mit 0,003 % Linolsäure angezogen wurden, weisen keine Fettsäuren mit einer Retensionszeit von Calendulasäure auf (nicht gezeigt). Ebenso enthalten die hydrolisierten Lipidextrakte von Hefezellen, die das FAD2-Gen exprimieren keine Calendulasäure (Fig. 3B).

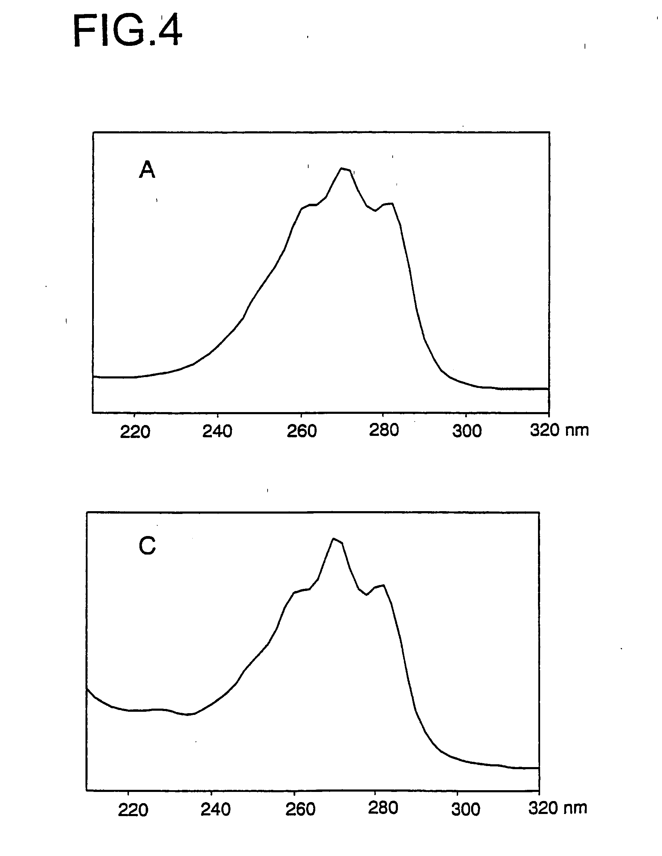

Die HPLC-Analyse der Extrakte von mit pYES2-CalDes transformierten Hefezellen, die mit 0,003 % Linolsäure angezogen wurden, hingegen zeigten ein Signal mit der Retensionszeit von Calendulasäure (Fig. 3C), das auch das gleiche Absorptionsspektrum mit einem Maximum bei 268 nm und Nebenmaxima bei 258 und 282 nm aufwies wie der Standard (Fig. 4A, Standard und C, Elutionsprofil von *S. cerevisiae* INCSv1 transformiert mit pYES2-CalDes aus Calendula officinalis). Damit war gezeigt, daß die Expression von Calendulasäure-Desaturase in Hefe zur Biosynthese von Calendulasäure führt. Der Nachweis von Calendulasäure aus transformierten Hefezellen gelang nur nach Hydrolyse der Lipide. In den freien Fettsäuren dieser Zellen konnte keine Calendulasäure nachgewiesen werden, das heißt Calendulasäure wird in Hefe in Lipide eingebaut. Da Hefe keine Triacylglyceride enthält, muß man davon ausgehen, daß die nachgewiesene Calendulasäure in den Phospholipiden der Hefe gebunden war.

Darüberhinaus enthalten die Lipidextrakte von transgenen Hefezellen, die gleichzeitig FAD2 und CalDes exprimieren ebenfalls Calendulasäure (nicht gezeigt).

### Beispiel 6: Expression der Calendulasäure-Desaturase in Arabidopsis thaliana und Linum usitatissimum

Die Expression der Calendulasäure-Desaturase aus Calendula officinalis in transgenen Pflanzen ist vorteilhaft, um den Calendulasäure-Gehalt in diesen Pflanzen zu erhöhen. Dazu wurde die CalDes cDNA in binäre Vektoren kloniert und über Agrobacterium-vermittelten DNA-Transfer in A. thaliana und L. usitatissimum übertragen. Die Expression der CalDes cDNA stand dabei unter der Kontrolle des konstitutiven CaMV 35 S-Promotors bzw, des samenspezifischen USP-Promotors.

Als Expressionsvektoren wurden der Vektor pBinAR (Höfgen und Willmitzer, Plant Science, 66, 1990: 221 - 230) bzw. das pBinAR Derivat pBinAR-USP, bei dem der CaMV 35 S-Promotor gegen den USP-Promotor aus V. faba ausgetauscht war, verwendet. Zur Umklonierung mußte die CalDes-cDNA aus dem Vektor pGEM-T ausgeschnitten werden. Dazu wurde zunächst mit NcoI geschnitten und mit Klenow zu glatten Enden aufgefüllt, anschließend wurde das Insert mit SalI herausgeschnitten und in die SmaI/SalI geschnittenen Vektoren pBinAR bzw. pBinAR-USP kloniert.

Die entstandenen Plasmide pBinAR-CalDes bzw. pBinAR-USP-CalDes wurden in Agrobacterium tumefaciens transformiert (Höfgen und Willmitzer, Nucl. Acids Res., 16, 1988: 9877). Die Transformation von A. thaliana erfolgte mittels "floral dip" (Clough und Bent, Plant Journal, 16, 1998: 735 - 743), die von L. usitatissimum durch Cokultivierung von Leinen-Hypokotylstücken mit transformierten A. tumefaciens Zellen.

Die Expression des CalDes-Gens in transgenen Arabidopsis und Linum Pflanzen wurde über Northern-Blot Analyse untersucht. Ausgewählte Pflanzen wurden auf ihren Gehalt an Calendulasäure im Samenöl untersucht.

Analog zum USP-Promotor kann auch der Napin-Promotor verwendet werden, um eine samenspezifische Expression von CalDes zu erreichen.

## Patentansprüche

1. Isolierte Nukteinsäure die für ein Polypeptid mit Desaturaseaktivität codiert, das Linolsäure zu Calendulasäure umsetzt, ausgewählt aus der Gruppe:
a) einer Nukleinsäure mit der in SEQ ID NO: 1 dargestellten Sequenz,
b) Nukleinsäure, die sich als Ergebnis des degenerierten genetischen Codes von der in SEQ ID NO: 1 dargestellten Nukleinsäure ableiten,
c) Derivate der in SEQ ID NO: 1 dargestellten Nukleinsäure die sich über Hybridisierung bei 42°C in 5 x SSC und 50 % Formamid isolieren lassen.

2. Aminosäuresequenz codiert durch eine Nukleinsäure gemäß Anspruch 1.

3. Aminosäuresequenz nach Anspruch 2, codiert durch die in SEQ ID NO: 1 dargestellte Nukleinsäure.

4. Nukleinsäurekonstrukt enthaltend eine Nukleinsäure gemäß Anspruch 1, wobei die Nukleinsäure mit einem oder mehreren Regulationssignalen verknüpft ist.

5. Vektor enthaltend eine Nukleinsäure gemäß Anspruch 1 oder ein Nukleinsäurekonstrukt gemäß Anspruch 4.

6. Nicht-humaner Organismus enthaltend mindestens ein Nukleinsäurekonstrukt gemäß Anspruch 4.

7. Nicht-humaner Organismus nach Anspruch 6, wobei es sich bei dem Organismus um eine Pflanze, einen Mikroorganismus oder ein Tier handelt.

8. Transgene Pflanze enthaltend ein funktionelles oder nicht funktionelles Nukleinsäurekonstrukt gemäß Anspruch 4.

9. Verfahren zur Herstellung von ungesättigten Fettsäuren **dadurch gekennzeichnet, daß** man mindestens eine Nukleinsäure gemäß Anspruch 1 oder mindestens ein Nukleinsäurekonstrukt gemäß Anspruch 4 in einen ÖI produzierenden nicht-humanen Organismus bringt, diesen Organismus anzieht und daß in dem Organismus enthaltene Öl isoliert und die im Öl enthaltenden Fettsäuren freisetzt.

10. Verfahren nach Anspruch 9, **dadurch gekennzeichnet, daß** es sich bei dem Organismus um eine Pflanze oder einen Mikroorganismus handelt.

11. Verwendung einer Nukleinsäure gemäß Anspruch 1 oder eines Nukleinsäurekonstrukt gemäß Anspruch 4 zur Herstellung von transgenen Pflanzen.

12. Verfahren nach Anspruch 9, **dadurch gekennzeichnet, dass** die im Verfahren hergestellten ungesättigten oder gesättigten Fettsäuren Nahrungsmitteln, Tierfutter, Kosmetika oder Pharmazeutika zugesetzt werden.

## Claims

1. An isolated nucleic acid which encodes a polypeptide with desaturase activity which converts linoleic acid into calendulic acid, selected from the following group:
a) a nucleic acid with the sequence shown in SEQ ID NO: 1,
b) nucleic acids which, as a result of the degeneracy of the genetic code, are derived from the nucleic acid shown in SEQ ID NO: 1,
c) derivatives of the nucleic acid shown in SEQ ID NO: 1 which can be isolated via hybridization at 42°C in 5 x SSC and 50% formamide.

2. The amino acid sequence encoded by the nucleic acid according to claim 1.

3. The amino acid sequence according to claim 2, encoded by the nucleic acid shown in SEQ ID NO: 1.

4. A nucleic acid construct comprising the nucleic acid according to claim 1, where the nucleic acid is linked to one or more regulatory signals.

5. A vector comprising the nucleic acid according to claim 1 or the nucleic acid construct according to claim 4.

6. A non-human organism comprising at least one nucleic acid construct according to claim 4.

7. The non-human organism according to claim 6, which is a plant, a microorganism or an animal.

8. A transgenic plant comprising the functional or nonfunctional nucleic acid construct according to claim 4.

9. A process for the preparation of unsaturated fatty acids, which comprises introducing at least one nucleic acid according to claim 1 or at least one nucleic acid construct according to claim 4 into an oil-producing non-human organism, growing this organism, isolating the oil comprised in the organism and liberating the fatty acids comprised in the oil.

10. The process according to claim 9, wherein the organism is a plant or a microorganism.

11. The use of the nucleic acid according to claim 1 or of the nucleic acid construct according to claim 4 for the generation of transgenic plants.

12. The process according to claim 9, wherein the unsaturated or saturated fatty acids prepared in the process are added to foodstuffs, animal feed, cosmetics or pharmaceuticals.

## Revendications

1. Acide nucléique isolé qui code pour un polypeptide à activité de désaturase qui convertit de l'acide linoléique en acide de calendula, choisi parmi le groupe :
a) d'un acide nucléique ayant la séquence représentée dans SEQ ID NO : 1,
b) des acides nucléiques qui, comme résultat du code génétique dégénéré, dérivent de l'acide nucléique représenté dans SEQ ID NO : 1,
c) des dérivés de l'acide nucléique représenté dans SEQ ID NO : 1 qu'on peut isoler par hybridation à 42°C dans 5 x SSC et 50 % de formamide.

2. Séquence d'acides aminés codée par un acide nucléique suivant la revendication 1.

3. Séquence d'acides aminés suivant la revendication 2, codée par l'acide nucléique représenté dans SEQ ID NO : 1.

4. Construction à base d'acide nucléique contenant un acide nucléique suivant la revendication 1, l'acide nucléique étant lié à un ou plusieurs signaux de régulation.

5. Vecteur contenant un acide nucléique suivant la revendication 1 ou une construction à base d'acide nucléique suivant la revendication 4.

6. Organisme non humain contenant au moins une construction à base d'acide nucléique suivant la revendication 4.

7. Organisme non humain suivant la revendication 6, pour lequel il s'agit, en ce qui concerne l'organisme, d'une plante, d'un micro-organisme ou d'un animal.

8. Plantes transgéniques contenant une construction à base d'acide nucléique fonctionnelle ou non fonctionnelle suivant la revendication 4.

9. Procédé de préparation d'acides gras insaturés, **caractérisé en ce qu'**on amène au moins un acide nucléique suivant la revendication 1 ou au moins une construction à base d'acide nucléique suivant la revendication 4 dans un organisme non humain produisant de l'huile, on cultive cet organisme et on isole l'huile contenue dans l'organisme et libère les acides gras contenus dans l'huile.

10. Procédé suivant la revendication 9, **caractérisé en ce que**, pour ce qui concerne l'organisme, il s'agit d'une plante ou d'un micro-organisme.

11. Utilisation d'un acide nucléique suivant la revendication 1 ou d'une construction à base d'acide nucléique suivant la revendication 4, pour la réalisation de plantes transgéniques.

12. Procédé suivant la revendication 9, **caractérisé en ce que** les acides gras insaturés ou saturés préparés dans le procédé sont ajoutés à des produits nutritifs, des aliments pour animaux, des cosmétiques ou des produits pharmaceutiques.
